(19) Europäisches Patentamt
European Patent Office
Office européen des brevets

(11) Veröffentlichungsnummer: **0 315 895 B1**

# (12) EUROPÄISCHE PATENTSCHRIFT

(45) Veröffentlichungstag der Patentschrift: **11.12.91**

(51) Int. Cl.⁵: **C07C 49/453**, C07C 35/37, C07C 69/14, C07C 69/06, A61K 7/46, C11B 9/00

(21) Anmeldenummer: **88118291.9**

(22) Anmeldetag: **03.11.88**

## (54) Tricyclische Verbindungen.

(30) Priorität: **12.11.87 CH 4425/87**

(43) Veröffentlichungstag der Anmeldung: **17.05.89 Patentblatt 89/20**

(45) Bekanntmachung des Hinweises auf die Patenterteilung: **11.12.91 Patentblatt 91/50**

(84) Benannte Vertragsstaaten: **CH DE ES FR GB IT LI NL**

(56) Entgegenhaltungen:
**EP-A- 0 029 259**
**US-A- 4 250 338**
**US-A- 4 368 128**

(73) Patentinhaber: **L. GIVAUDAN & CIE Société Anonyme**
**CH-1214 Vernier-Genève(CH)**

(72) Erfinder: **Fráter, Georg, Dr.**
**Turicumstrasse 5**
**CH-8610 Uster(CH)**
Erfinder: **Müller, Urs**
**Im Grünenhof 25**
**CH-8625 Gossau(CH)**

(74) Vertreter: **Urech, Peter, Dr. et al**
**Grenzacherstrasse 124 Postfach 3255**
**CH-4002 Basel(CH)**

**EP 0 315 895 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Riechstoffe. Es handelt sich dabei um die Verbindungen der Formel

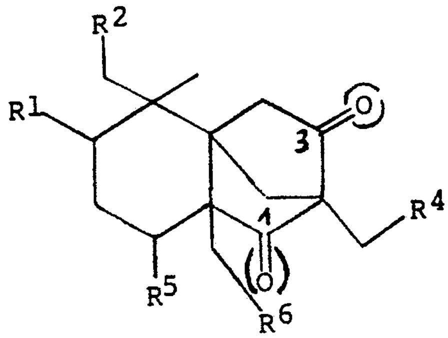

I

worin die Symbole $R^1$, $R^2$, $R^4$, $R^5$ und $R^6$ Wasserstoff oder Methyl darstellen, wobei nur eines der Symbole $R^1$, $R^2$ und $R^4$ Methyl sein kann, und im Falle $R^5$ und/oder $R^6$ Methyl bedeutet, $R^1$, $R^2$ und $R^4$ Wasserstoff darstellen und worin die Klammern ( ) bedeuten, dass jeweils nur eine Ketogruppe im Molekül vorhanden ist, und wobei im Falle der 1-Ketogruppe das Kohlenstoffatom 3 auch mono-methylsubstituiert sein kann.

Die Formel I beinhaltet also die Hexahydro-2H-2,4a--methanonaphthalin-1(5H)-one

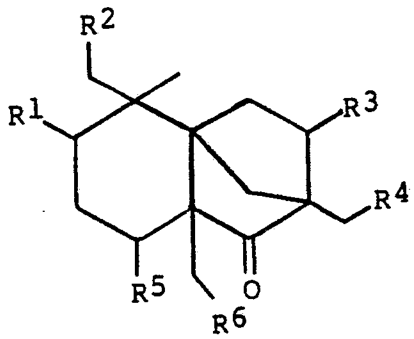

Ia

und die Hexahydro-2H-2,4a-methanonaphthalin-3(4H)-one

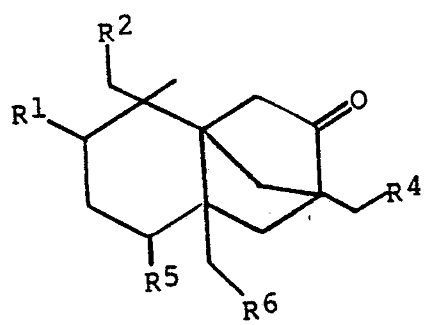

Ib

worin $R^1$, $R^2$, $R^4$, $R^5$ und $R^6$ obige Bedeutungen besitzen und $R^3$ Wasserstoff oder Methyl sein kann.

Die Erfindung betrifft ferner ein Verfahren zur Herstellung der Verbindungen I.

Dieses Verfahren ist dadurch gekennzeichnet, dass man eine Verbindung der Formel

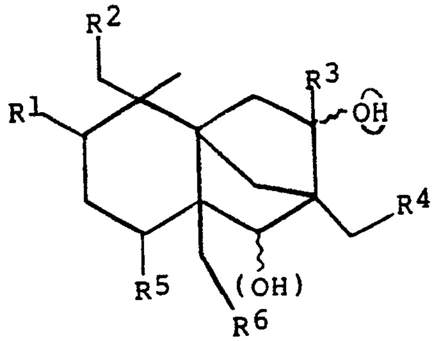

II

worin $R^1$ bis $R^6$ obige Bedeutung besitzen und die Klammern ( ) bedeuten, dass jeweils nur eine Hydroxygruppe im Molekül vorhanden ist,
oxydiert.

Die Oxydation kann nach den an sich bekannten Methoden der Oxydation sekundärer Alkohole zu Ketonen durchgeführt werden; geeignete Oxydationsmittel sind demgemäss Chromsäure bzw. das Jones-Reagens, Javellewasser, insbesondere aber $H_2O_2$, vorzugsweise in Anwesenheit von Ammonmolybdat als Katalysator. Man arbeitet im letzteren Fall vorzugsweise in Anwesenheit von Phasentransferkatalysatoren, wie z.B. in Anwesenheit von Tetra-n-alkyl-ammoniumhalogeniden, und bei Temperaturen von ca. 20-100 °C.

Die Auftrennung des üblicherweise erhaltenen Isomerengemisches I kann durch übliche Methoden, insbesondere Chromatographie und/oder Umkristallisieren erfolgen. Sie ist aber aus wirtschaftlichen Gründen nicht attraktiv.

Die Herstellung des Ausgangsmaterials II kann gemäss dem folgenden Reaktionsschema bewerkstelligt werden. Die Verbindungen II und III sind neu und bilden einen weiteren Gegenstand der vorliegenden Erfindung.

## Reaktionsschema

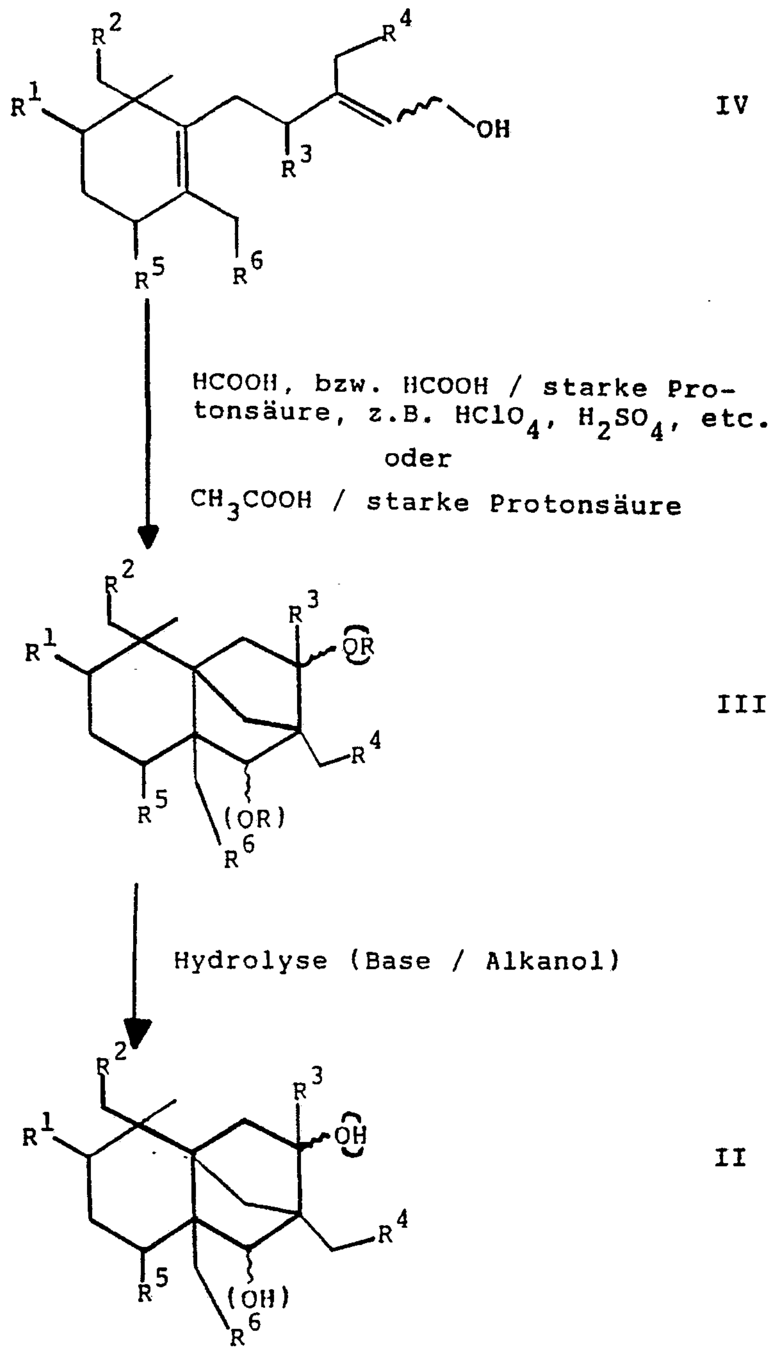


IV

III

II

R = Formyl, Acetyl

Das Verhältnis HCOOH/starke Protonsäure bzw. $CH_3$ COOH/starke Protonsäure bestimmt im wesentlichen das Verhältnis Ia:Ib. Im allgemeinen gilt: Je mehr Protonsäure, je mehr Verbindung Ib wird gebildet.

Es wird in Abhängigkeit der eingesetzten Säuren das zusätzliche Auftreten eines weiteren, ebenfalls geruchsaktiven tricyclischen Ketons

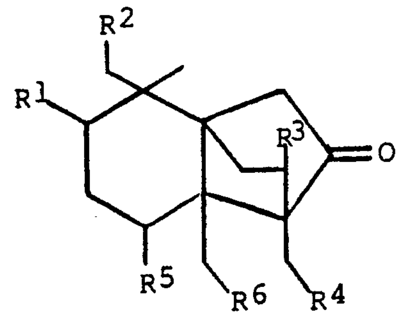

V

worin die Reste $R^1$ bis $R^6$ obige Bedeutung besitzen, festgestellt. Diese Verbindung kann deshalb mit dem üblicherweise anfallenden erfindungsgemässen Gemisch der Verbindungen I mitverwendet werden.

Die Verbindungen der Formeln II und III kommen infolge ihrer tricyclischen Struktur als Exo- und als Endoisomere vor. Die vorliegende Erfindung soll diese beiden isomeren Formen umfassen.

Die Verbindungen der Formel I zeichnen sich durch sehr kräftige, diffusive und sehr natürlich-warme, holzige Noten in Richtung Vetiver aus. Erwähnenswert ist ferner ihre Beständigkeit gegenüber Oxydationsmitteln und ihre Unempfindlichkeit gegenüber schwachen Säuren und Basen.

Die Erfindung betrifft demgemäss auch die Verwendung der Verbindungen I als Riechstoffe.

Die Verbindungen der Formel I eignen sich aufgrund ihrer natürlichen Geruchsnoten insbesondere zur Modifizierung von bekannten Kompositionen. Hervorgehoben werden soll insbesondere ihre ausserordentliche Geruchsstärke: Der Geruchsschwellenwert beträgt ca. 0,15 ng/l, der Geruchswert 60 000--300 000. Bezüglich Definition von Geruchswert und Geruchsschuellenwert siehe z.B. Ulrich A. Huber, Seifen - Oele - Fette - Wachse 110, Nr. 15 (1984) 448-451.

So beträgt der Geruchsschwellenwert der Verbindung Ia gemäss Beispiel 1 0,36 ng/l, der Geruchsschwellenwert des Desmethylhomologen des Beispiels VII der US-PS 4.250 338 (= Beispiel 7 der US-PS 4.368 128) andererseits 9,46 ng/l.

Die Verbindungen I verbinden sich mit zahlreichen bekannten Riechstoffingredientien natürlichen oder synthetischen Ursprungs, wobei die Palette der natürlichen Riechstoffe sowohl leicht- als auch mittel- und schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl Mandarinenöl, etc.), Mastix-Absolue, Myrtenöl, Palmarosaöl, Patchouliöl, Petitgrainöl Paraguay, Wermutöl,
- Alkohole, wie Farnesol, Geraniol, Linalool, Nerol, Phenyläthylalkohol, Rhodinol, Zimtalkohol, Sandalore (3-Methyl-5-(2,2,3-trimethylcyclopent -3-en-1-yl)pentan--2-ol),
- Aldehyde, wie Citral, Helional®, α-Hexylzimtaldehyd, Hydroxycitronellal, Lilial® (p-tert.Butyl-α-methyl--dihydrozimtaldehyd), Methylnonylacetaldehyd,
- Ketone, wie Allyljonon, α-Jonon, β-Jonon, Isoraldein (Isomethyl-α-ionon), Methyljonon,
- Ester, wie Allyl-phenoxyacetat, Benzyl-salicylat, Cinnamylpropionat, Citronellyl-acetat, Citronellyl--äthoxolat (Citronellyl . O - CO -CO . $OC_2H_5$), Decyl-acetat, Dimethylbenzylcarbinyl-acetat, Dimethylbenzylcarbinyl-butyrat, Aethyl-acetoacetat, Aethyl--acetylacetat, Hexenyl-isobutyrat, Linalylacetat, Methyl-dihydrojasmonat, Styrallylacetat, Vetiveryl-acetat, 2-Aethyl-6,6-dimethyl (und 2,3,6,6-Tetramethyl)--2-cyclohexen-1-carbonsäureäthylester ("Givescone"),
- Lactone, wie γ-Undecalacton,
- verschiedene, in der Parfümerie oft benützte Komponenten, wie Ketonmoschus, Indol, p-Menthan-8-thiol-3-on, Methyleugenol.

Bemerkenswert ist ferner die Art und Weise, wie die Verbindungen I die Geruchsnoten bekannter Kompositionen abrunden und harmonisieren, ohne aber in unangenehmer Weise zu dominieren.

Die Verbindungen der Formel I (bzw. deren Gemische) lassen sich in weiten Grenzen einsetzen, die beispielsweise von 0,1 (Detergentien) - 20% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 1 und ca. 10%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen 1 können demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführtne bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Im Vordergrund des Interesses ist die Verbindung I mit $R^1 = R^2 = R^3 = R^4 = R^5 = R^6 = H$, mit holzigen, Vetiver-, pfeffrigen, Rosen- und leicht fruchtigen Noten.

Die Geruchsnoten der entsprechenden Verbindung Ia sind: holzig, agreste, mit einem fruchtigen Aspekt; die Geruchsnoten von Ib sind: holzig, kamphrig, Kiefer, cedrig, mit Tabak- und fettigen Aspekten; von V: holzig, kamphrig, erdig, Borneol.

Weitere interessante Verbindungen sind

a) die entsprechenden aus Dihydro-β-iron zugänglichen Verbindungen I mit $R^1 = CH_3$ und $R^2 = R^3 = R^4 = R^5 = R^6 = H$,

b) die entsprechenden aus Dihydro-β-raldein zugänglichen Verbindungen I mit $R^4 = CH_3$ und $R^1 = R^2 = R^3 = R^5 = R^6 = H$.

## Beispiel 1

a) In einem 4,5 -Sulfierkolben, der mit Rückflusskühler, Thermometer, Tropftrichter, Gaseinleitungsrohr und mechanischem Rührer versehen ist, werden 2 l Ameisensäure (98%) vorgelegt und nun bei Zimmertemperatur 444 g des Alkohols IV ($R^1 = R^2 = R^3 = R^4 = R^5 = R^6 = H$) (2 Mol) innerhalb 3 Minuten zugetropft, wobei die Temperatur ca. 2°C ansteigt und eine grau-braune Lösung entsteht. Es wird 45 Minuten bei 40-45°C weitergerührt. Das Edukt verschwindet komplett und durch gaschromatographische Analyse wird das Entstehen des Formiates III festgestellt. Dieses Gemisch wird mit Wasser/Eis versetzt, mit Hexan ausgeschüttelt, neutral gewaschen, getrocknet und eingedampft; es entstehen 542 g Rohprodukt. Dieses Rohprodukt wird über eine 5 cm-Widmerkolonne fraktioniert destilliert und die Fraktion 2 vom Siedepunkt 90-120°C/0,07 mmHg, welche Fraktion 252 g wiegt, weiterverwendet.

b) 250 g Formiat III ex Stufe a) (1 Mol) werden bei 30°C zu 100 g 85%ige KOH in 400 ml Methanol und 100 ml Wasser getropft, wobei die Temperatur des Gemisches auf 63°C ansteigt; das Gemisch siedet leicht. Es wird 30 Minuten bei 65°C Wasserbadtemperatur gerührt, abgekühlt und auf 2 l Eiswasser gegossen; es wird dreimal mit Hexan und 20% t-Butyl-methyläther ausgeschüttelt, getrocknet und eingedampft, wobei 260 g Rohprodukt anfallen. Dieses Rohprodukt wird am Hochvakuum über eine 10 cm-Widmerkolonne destilliert und die Fraktion vom Siedepunkt 93-115°C/0,06 mmHg, welche Fraktion 213,5 g wiegt, weiter verwendet. Es handelt sich um ein stark viskoses Oel, welches beim Stehen wachsartig erstarrt.

$c_1$) In einem 1 l-Dreihalskolben, der mit Thermometer, Tropftrichter, Kühler und Rührer versehen ist, werden 150 g (0.68 Mol) des Alkohols II der Stufe b) mit 30%-igem $H_2O_2$ gemäss B.M. Trost et al., Tetr. Letters 25 (2), 173-176 (1984) oxydiert. Es fallen 159 g blau-graues Oel an. Diese werden nun einer Hochvakuumdestillation unterworfen; die Fraktion vom Siedepunkt 85-87°C/0,09 mmHg stellt ein Produkt von olfaktisch guter Qualität dar. Das Verhältnis von Keton Ia zu Keton Ib beträgt 7:2; dieses Ketongemisch enthält zudem 10% des Ketons V mit $R^1$ bis $R^6$ = Wasserstoff. Die Auftrennung in die Komponenten erfolgt durch Chromatographie mittels Hexan/Aether an Kieselgel.

## Physikalische Daten

Keton Ia: $^{13}C$-NMR ($CDCl_3$):

222,9 (s,C-1), 53,9 (s,C-4a), 53,3 (s,C-2), 49,7 (s,C-8a), 42,4 (t,C-9), 38,99 (t,C-6), 32,78 (s,C-5), 32,44 (t,C-3), 30,86 (t,C-8), 27,55 (q,$CH_3$-C-5), 25,46 (t,C-4), 24,56 (q,$CH_3$-C-5), 19,49 (q,$CH_3$-C-8a), 18,01 (t,C-7), 14,82 (q,$CH_3$-C-2).

Keton Ib: $^{13}C$-NMR ($CDCl_3$):

216,53 (s,C-2), 60,54 (s,C-1), 52,30 (s,C-3a), 47,85 (s,C-7a), 45,15 (t,C-3), 36,01 (t,C-5), 33,60 (s,C-4), 29,90 (t,C-8), 28,73 (q,$CH_{3,eq}$-C-4), 28,69 (t,C-7), 27,28 (t,C-9), 23,42 (q,$CH_{3,ax}$-C-4), 18,73 (t,C-6), 15,33 (q,$CH_3$-C-7a), 9,26 (q,$CH_3$-C-1).

Keton V: $^{13}C$-NMR ($CDCl_3$):

215,0 (s,C-3), 53,66 (s,C-2), 53,28 (s,C-4a), 52,20 (t,C-1), 44,09 (t,C-9), 42,21 (t,C-4), 40,89 (t,C-8), 39,84 (s,C-8a), 38,23 (t,C-6), 33,09 (s,C-5), 29,19 (q,$CH_{3eq}$-C-5), 25,23 (q,$CH_{3ax}$-C-5), 24,55 (q,$CH_3$-C-8a), 19,61 (t,C-7), 14,58 (q,$CH_3$-C-2).

$c_2$) In einem 750 ml-Sulfierkolben, der mit Thermometer, Tropftrichter und Rührer versehen ist und in

einem Eisbad steht, werden 44 g Alkoholgemisch der Stufe b) (0,2 Mol) in 200 ml Eisessig gelöst und bei 15-20° C unter Kühlen tropfenweise mit 90 ml Javellewasser (13-14% aktives Chlor) versetzt, wobei die gelbe Phase am Schluss des Zutropfens 5 Minuten bestehen bleibt. Es wird mit Wasser und Aether/Hexan = 2:8 aufgearbeitet, hierauf mit Wasser, dann mit $NaHSO_3$-Lösung gewaschen und mit IN Kalilauge ausgeschüttelt, das Produkt wird getrocknet und eingedampft, wobei 50,2 g Rohprodukt anfallen. Destillation am Hochvakuum liefert nach einem Vorlauf die Fraktion vom Siedepunkt 86-88° C/0,08 mmHg (36,4 g) die nochmals rektifiziert wird. Es entstehen 34,9 g farbloses Oel I vom Siedepunkt 80-81° C/0,06 mmHg.

$c_3$) In einem 1 l-Zweihalskolben, ausgerüstet mit Thermometer, Tropftrichter und Magnetrührer, und der in einem Eis/NaCl-Bad steht, werden unter Kühlen mittels Eis/Kochsalz-Bad 51 g (0,232 Mol) Alkohol II in 300 ml Aceton gelöst und bei 5-10° C mit 85 ml Jones-Reagens versetzt, bis eine leichte orange Färbung bestehen bleibt. Es wird 1 Stunde bei Raumtemperatur nachgerührt und auf 1 l Eis/Wasser gegossen. Die wässrige Schicht wird dreimal mit 150 ml Hexan ausgeschüttelt, mit $K_2CO_3$-Lösung gewaschen, getrocknet und eingedampft, wobei 56 g öliges Rohprodukt I anfallen. Dieses wird am Hochvakuum destilliert. Es resultieren 46 g farbloses Oel I vom Siedepunkt 85-90° C/0,1 mmHg.

Beispiel 2

In den nachfolgenden Formulierungen steht "Verbindung I" für ein Gemisch von Ia:Ib:V = 7:2:1.

a) Base Richtung blumig-orientalisch

| | Gewichtsteile |
|---|---|
| Geraniumoxyd (Rosenoxyd) | 1 |
| Dimetol | 9 |
| Citronellol extra | 10 |
| Givescone® | 10 |
| Methyl-anthranilat extra | 10 |
| Geranylacetat | 20 |
| Cumarin rein krist. | 20 |
| Vanillin | 20 |
| Canthoxal (3-(4'-Methoxy-phenyl)-2-methyl-propanal | 20 |
| Sandelholzessenz Mysore | 30 |
| Eugenol extra | 30 |
| Phenyläthylalkohol | 40 |
| Ylang-Ylang-öl | 40 |
| natürliches, gereinigtes Aurantiol | 40 |
| Novalid (1,4,6,6,8,8-Hexamethyl-as-hydrindacen-3-on | 50 |
| Heliotropin krist. | 50 |
| Verbindung I | 50 |
| Ketonmoschus | 60 |
| Benzylacetat extra | 80 |
| Linalool synthet. | 80 |
| Hydroxycitronellal | 100 |
| Methyldihydrojasmonat | 110 |
| Lyral | 120 |
| | 1000 |

Die Verbindungen I verleihen obiger Komposition Volumen, Stärke und Körper. Die würzige Eugenol-Note wird verstärkt.

b) Base Richtung Hesperiden-holzig-Moschus

| | Gewichtsteile |
|---|---|
| Acetanisol | 10 |
| Eugenol extra | 10 |
| Zedernblättöl | 10 |
| Anethol aus Sternanisöl | 10 |
| Estragol | 10 |
| Phenyläthylalkohol | 20 |
| Citronellol extra | 30 |
| Lilial P® | 40 |
| Sandalore ® | 40 |
| Linalool synthet. | 40 |
| Musc Moskene | 40 |
| Orangenöl Florida | 60 |
| Benzylacetat extra | 60 |
| Linalylacetat synthet. | 60 |
| Benzylsalicylat | 100 |
| Novalid | 120 |
| α-Hexylzimtaldehyd | 140 |
| Verbindung I | 200 |
| | 1000 |

Die Verbindung I erbringt in dieser Base Stärke, Fülle und Volumen.

c) Base Richtung blumig (Rose, Veilchen)-grün

| | Gewichtsteile |
|---|---|
| 2,6-Nonadienal (1% Diprocol) | 2 |
| Galbanum-öl natürl. | 3 |
| Eugenol extra | 5 |
| 4-Isopropyl-cyclohexanol | 10 |
| cis-3-Hexenol (10% Diprocol) | 10 |
| Phenylacetaldehyd (10% Diprocol) | 10 |
| Verbindung I | 20 |
| Musc moskene | 20 |
| Zedernholzöl Virginia | 20 |
| Methyloctincarbonat (10% Diprocol) | 20 |
| Givescone ® | 20 |
| Geranium-öl Bourbon | 50 |
| Lilial P ® | 50 |
| Benzylacetat extra | 50 |
| α-Irison | 50 |
| Methyldihydrojasmonat | 50 |
| p-tert.Butylcyclohexylacetat | 80 |
| Geraniol extra | 80 |
| Citronellol extra | 150 |
| Phenyläthylalkohol | 300 |
| | 1000 |

Die Verbindung I verstärkt die Maiglöckchennote in eindeutiger Weise. Die Verbindung I erbringt ganz generell Volumen, Diffusion, Natürlichkeit, Harmonie, Stärke.

## Patentansprüche

1. Verbindungen der Formel

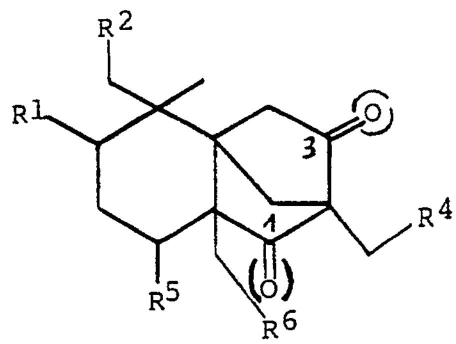

I

worin die Symbole $R^1$, $R^2$, $R^4$, $R^5$ und $R^6$ Wasserstoff oder Methyl darstellen, wobei nur eines der Symbole $R^1$, $R^2$ und $R^4$ Methyl sein kann, und im Falle $R^5$ und/oder $R^6$ Methyl bedeutet, $R^1$, $R^2$ und $R^4$ Wasserstoff darstellen und worin die Klammern ( ) bedeuten, dass jeweils nur eine Ketogruppe im Molekül vorhanden ist, und wobei im Falle der 1-Ketogruppe das Kohlenstoffatom 3 auch monomethylsubstituiert sein kann.

**2.** Verbindungen der Formel I gemäss Anspruch 1 mit der Formel

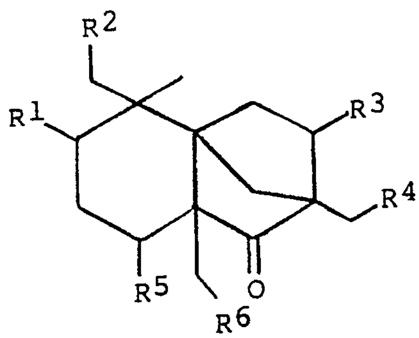

Ia

worin $R^1$ bis $R^6$ Wasserstoff oder Methyl darstellen, wobei nur eines der Symbole $R^1$ bis $R^4$ Methyl sein kann, und im Falle $R^5$ und/oder $R^6$ Methyl bedeutet, $R^1$ bis $R^4$ Wasserstoff darstellen.

**3.** Hexahydro-2,5,5,8a-tetramethyl-2H-2,4a-methano-naphthalin-1(5H)-on als Verbindung gemäss Anspruch 2.

**4.** Verbindungen der Formel I gemäss Anspruch 1 mit der Formel

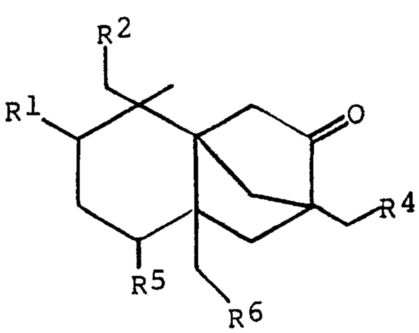

Ib

worin $R^1$, $R^2$, $R^4$, $R^5$ und $R^6$ Wasserstoff oder Methyl darstellen, wobei nur eines der Symbole $R^1$, $R^2$ und $R^4$ Methyl sein kann, und im Falle $R^5$ und/oder $R^6$ Methyl bedeutet, $R^1$, $R^2$ und $R^4$ Wasserstoff darstellen.

5. Hexahydro-2,5,5,8a-tetramethyl-2H-2,4a-methano-naphthalin-3(4H)-on als Verbindung gemäss Anspruch 4.

6. Verbindungen der Formel

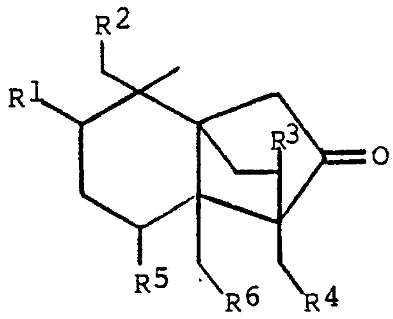

V

worin $R^1$ bis $R^6$ Wasserstoff oder Methyl darstellen, wobei nur eines der Symbole $R^1$ bis $R^4$ Methyl sein kann, und im Falle $R^5$ und/oder $R^6$ Methyl bedeutet, $R^1$ bis $R^4$ Wasserstoff darstellen.

7. 1,7a-trans-Hexahydro-1,4,4,7a-tetramethyl-1,3a-äthano-3aH-inden-2(3H)-on als Verbindung gemäss Anspruch 6.

8. Verbindungen der Formel

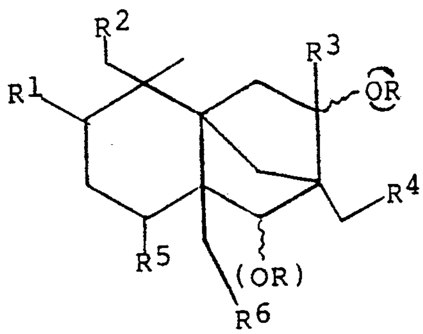

III'

worin die Symbole $R^1$ bis $R^6$ Wasserstoff oder Methyl darstellen, wobei nur eines der Symbole $R^1$ bis $R^4$ Methyl sein kann, und im Falle $R^5$ und/oder $R^6$ Methyl bedeutet, $R^1$, $R^2$, $R^3$ und $R^4$ Wasserstoff darstellen und worin R für Wasserstoff, Formyl oder Acetyl steht und die Klammern ( ) bedeuten, dass jeweils nur ein Rest OR im Molekül vorhanden ist.

9. Riechstoffkomposition, gekennzeichnet durch einen Gehalt an einer Verbindung der Formel

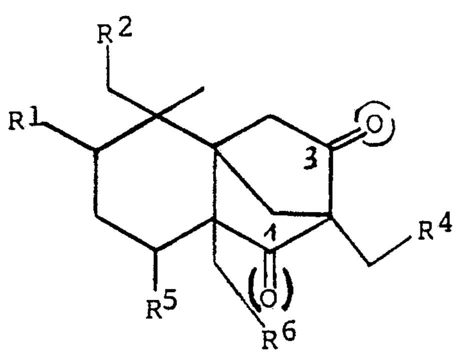

I

worin die Symbole $R^1$, $R^2$, $R^4$, $R^5$ und $R^6$ Wasserstoff oder Methyl darstellen, wobei nur eines der Symbole $R^1$, $R^2$ und $R^4$ Methyl sein kann, und im Falle $R^5$ und/oder $R^6$ Methyl bedeutet, $R^1$, $R^2$ und $R^4$ Wasserstoff darstellen und worin die Klammern ( ) bedeuten, dass jeweils nur eine Ketogruppe im Molekül vorhanden ist, und wobei im Falle der 1-Ketogruppe das Kohlenstoffatom 3 auch monomethylsubstituiert sein kann.

**10.** Riechstoffkomposition gemäss Anspruch 9, gekennzeichnet durch einen Gehalt an einer Verbindung I mit $R^1 = R^2 = R^4 = R^5 = R^6$ = Wasserstoff.

**11.** Verfahren zur Herstellung der Verbindungen der Formel

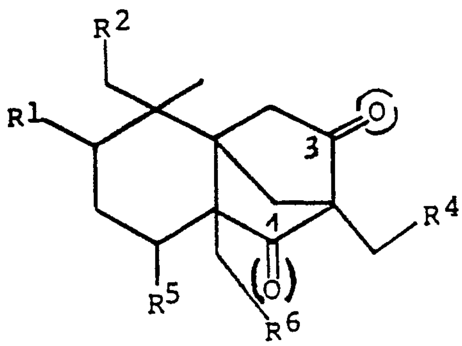

I

worin die Symbole $R^1$, $R^2$, $R^4$, $R^5$ und $R^6$ Wasserstoff oder Methyl darstellen, wobei nur eines der Symbole $R^1$, $R^2$ und $R^4$ Methyl sein kann, und im Falle $R^5$ und/oder $R^6$ Methyl bedeutet, $R^1$, $R^2$ und $R^4$ Wasserstoff darstellen und worin die Klammern ( ) bedeuten, dass jeweils nur eine Ketogruppe im Molekül vorhanden ist, und wobei im Falle der 1-Ketogruppe das Kohlenstoffatom 3 auch monomethylsubstituiert sein kann,
dadurch gekennzeichnet, dass man eine Verbindung der Formel

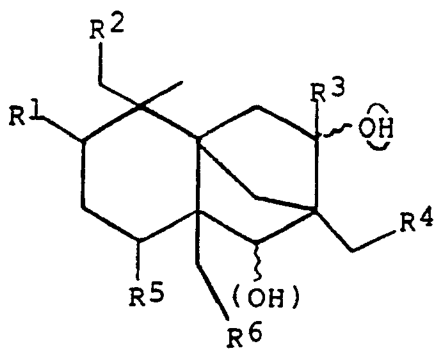

II

worin die Klammern ( ) bedeuten, dass jeweils nur eine Hydroxygruppe im Molekül vorhanden ist, und $R^3$ Wasserstoff oder Methyl ist,
oxydiert.

**12.** Verfahren nach Anspruch 11, dadurch gekennzeichnet, dass man eine Verbindung der Formel II mit $R^1 = R^2 = R^3 = R^4 = R^5 = R^6$ = Wasserstoff oxydiert.

**13.** Verfahren zur Herstellung von Riechstoffkompositionen, dadurch gekennzeichnet, dass man eine Verbindung I gemäss Anspruch 1 mit den üblichen Formulierungshilfsstoffen und gegebenenfalls mit andern Riechstoffen zusammenbringt.

**14.** Verwendung von Verbindungen der Formel

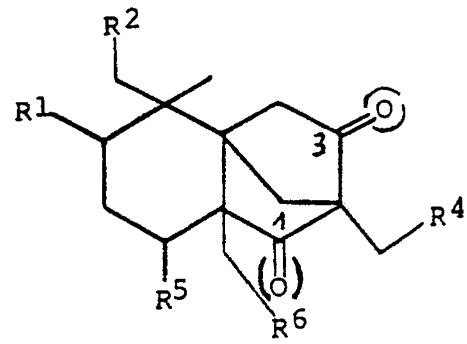

I

worin die Symbole $R^1$, $R^2$, $R^4$, $R^5$ und $R^6$ Wasserstoff oder Methyl darstellen, wobei nur eines der Symbole $R^1$, $R^2$ und $R^4$ Methyl sein kann und im Falle $R^5$ und/oder $R^6$ Methyl bedeutet, $R^1$, $R^2$ und $R^4$ Wasserstoff darstellen und worin die Klammern ( ) bedeuten, dass jeweils nur eine Ketogruppe im Molekül vorhanden ist, und wobei im Falle der 1-Ketogruppe das Kohlenstoffatom 3 auch mono-methylsubstituiert sein kann,
als Riechstoffe.

**Claims**

**1.** Compounds of the formula

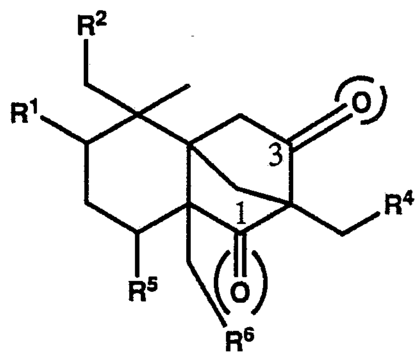

I

wherein the symbols $R^1$, $R^2$, $R^4$, $R^5$ and $R^6$ represent hydrogen or methyl, with the proviso that only one of the symbols $R^1$, $R^2$ and $R^4$ can be methyl and, where $R^5$ and/or $R^6$ signifies methyl, $R^1$, $R^2$ and $R^4$ represent hydrogen, and wherein the brackets ( ) signify that only one keto group is present in the molecule, and wherein the carbon atom 3 can also be mono-methyl substituted when the 1-keto group is present.

**2.** Compounds of formula 1 in accordance with claim 1 having the formula

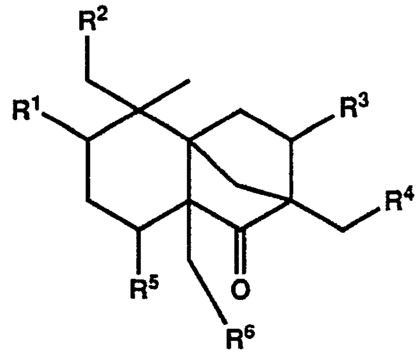

Ia

wherein $R^1$ to $R^6$ represent hydrogen or methyl, with the proviso that only one of the symbols $R^1$ to $R^4$ can be methyl and, where $R^5$ and/or $R^6$ signifies methyl, $R^1$ to $R^4$ represent hydrogen.

3. Hexahydro-2,5,5,8a-tetramethyl-2H-2,4a-methano-naphthalen-1(5H)-oneas the compound in accordance with claim 2.

4. Compounds of formula I in accordance with claim 1 having the formula

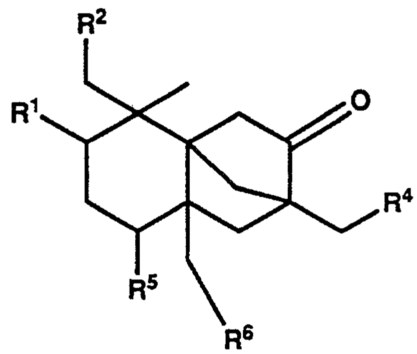

Ib

wherein $R^1$, $R^2$, $R^4$, $R^5$ and $R^6$ represent hydrogen or methyl, with the proviso that only one of the symbols $R^1$, $R^2$ and $R^4$ can be methyl and, where $R^5$ and/or $R^6$ signifies methyl, $R^1$, $R^2$ and $R^4$ represent hydrogen.

5. Hexahydro-2,5,5,8a-tetramethyl-2H-2,4a-methano-naphthalen-3(4H)-oneas the compound in accordance with claim 4.

6. Compounds of the formula

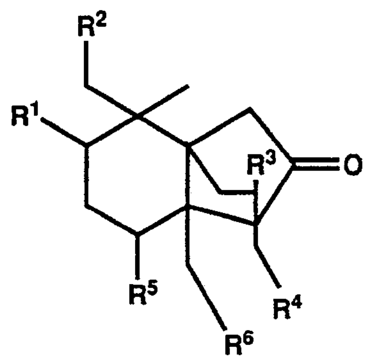

V

wherein $R^1$ to $R^6$ represent hydrogen or methyl, with the proviso that only one of the symbols $R^1$ to $R^4$ can be methyl and, where $R^5$ and/or $R^6$ signifies methyl, $R^1$ to $R^4$ represent hydrogen.

**7.** 1,7a-trans-Hexahydro-1,4,4,7a-tetramethyl-1,3a-ethano-3aH-inden-2(3H)-one as the compound in accordance with claim 6.

**8.** Compounds of the formula

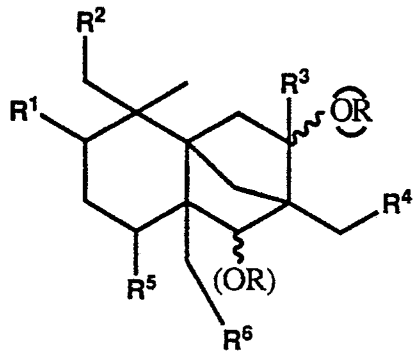

III

wherein the symbols $R^1$ to $R^6$ represent hydrogen or methyl, with the proviso that only one of the symbols $R^1$ to $R^4$ can be methyl and, where $R^5$ and/or $R^6$ signifies methyl, $R^1$, $R^2$, $R^3$ and $R^4$ represent hydrogen, and wherein R stands for hydrogen, formyl or acetyl and the brackets ( ) signify that only one OR residue is present in the molecule.

**9.** An odorant composition, characterized in that it contains a compound of the formula

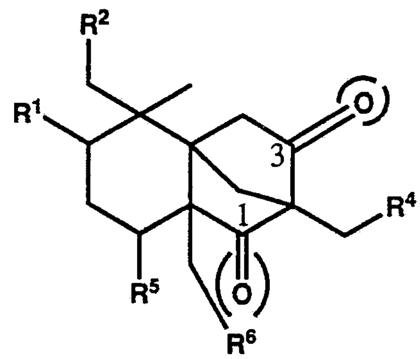

I

wherein the symbols $R^1$, $R^2$, $R^4$, $R^5$ and $R^6$ represent hydrogen or methyl, with the proviso that only one of the symbols $R^1$, $R^2$ and $R^4$ can be methyl and, where $R^5$ and/or $R^6$ signifies methyl, $R^1$, $R^2$, and $R^4$ represent hydrogen, and wherein the brackets ( ) signify that only one keto group is present in the molecule, and wherein the carbon atom 3 can also be mono-methyl substituted when the 1-keto group is present.

**10.** An odorant composition in accordance with claim 9, characterized in that it contains a compound with $R^1 = R^2 = R^3 = R^4 = R^5 = R^6 =$ hydrogen.

**11.** A process for the manufacture of the compounds of the formula

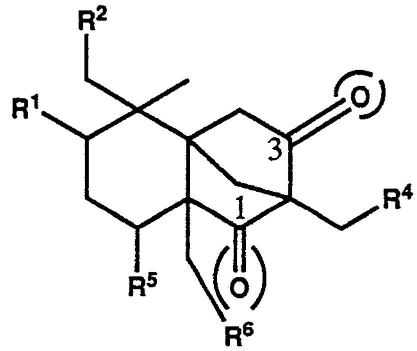

I

wherein the symbols $R^1$, $R^2$, $R^4$, $R^5$ and $R^6$ represent hydrogen or methyl, with the proviso that only one of the symbols $R^1$, $R^2$ and $R^4$ can be methyl and, where $R^5$ and/or $R^6$ signifies methyl, $R^1$, $R^2$ and $R^4$ represent hydrogen, and wherein the brackets ( ) signify that only one keto group is present in the molecule, and wherein the carbon atom 3 can also be mono-methyl substituted when the 1-keto group is present,
characterized by oxidizing a compound of the formula

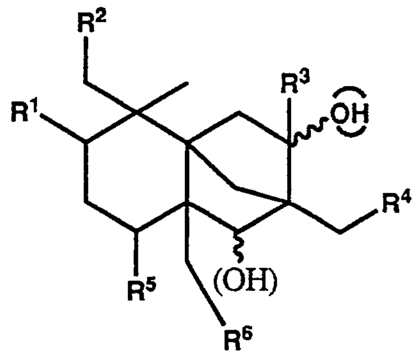

II

wherein the brackets ( ) signify that only on hydroxy group is present in the molecule and $R^3$ is hydrogen or methyl.

**12.** A process according to claim 11, characterized in that a compound of formula II with $R^1 = R^2 = R^3 = R^4 = R^5 = R^6 =$ hydrogen is oxidized.

**13.** A process for the manufacture of odorant compositions, characterized by bringing together a compound I in accordance with claim 1 with usual formulation adjuvants and, if desired, with other odorants.

**14.** The use of compounds of the formula

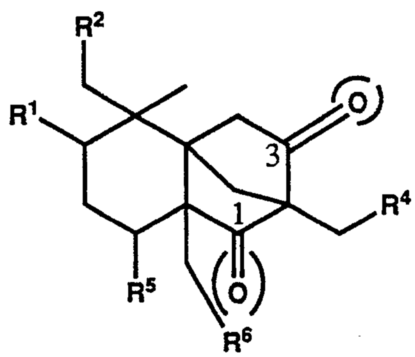

I

wherein the symbols $R^1$, $R^2$, $R^4$, $R^5$ and $R^6$ represent hydrogen or methyl, with the proviso that only one of the symbols $R^1$, $R^2$ and $R^4$ can be methyl and, where $R^5$ and/or $R^6$ signifies methyl, $R^1$, $R^2$ and $R^4$ represent hydrogen, and wherein the brackets ( ) signify that only one keto group is present in the molecule, and wherein the carbon atom 3 can also be mono-methyl substituted when the 1-keto group is present,
as odorants.

**Revendications**

1. Composés de formule :

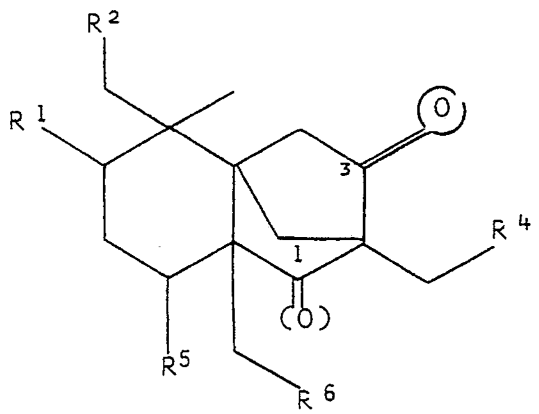

I

où les symboles $R^1$, $R^2$, $R^4$ , $R^5$ et $R^6$ représentent l'hydrogène ou le méthyle, l'un des symboles $R^1$ , $R^2$ et $R^4$ seulement pouvant être le méthyle et, dans le cas ou $R^5$ et/ou $R^6$ représentent le méthyle, $R^1$ , $R^2$ et $R^4$ représentent l'hydrogène, les parenthèses () signifient qu'un groupe céto seulement est présent dans la molécule et, dans le cas d'un groupe céto en 1, l'atome de carbone en 3 peut être également monométhyle substitué.

2. Composés de formule I selon la revendication 1 ayant la formule :

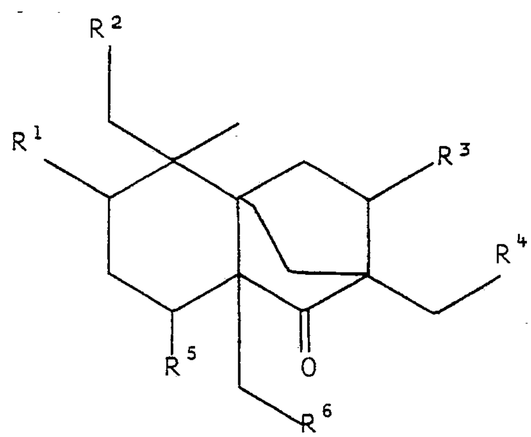

Ia

où $R^1$ à $R^6$ représentent l'hydrogène ou le méthyle, l'un des symboles $R^1$ à $R^4$ seulement pouvant être le méthyle, et dans le cas où $R^5$ et/ou $R^6$ représentent le méthyle, $R^1$ à $R^4$ représentent l'hydrogène.

3. La hexahydro-2,5,5,8a-tétraméthyl-2H-2,4a-méthano-naphtalèn-1(5H)-one comme composé selon la revendication 2.

4. Composés de formule I selon la revendication 1 ayant la formule :

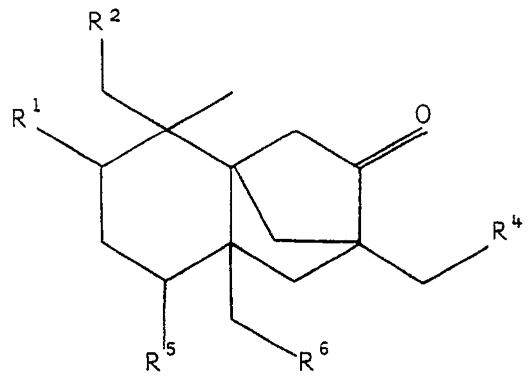

Ib

où $R^1$, $R^2$, $R^4$, $R^5$ et $R^6$ représentent l'hydrogène ou le méthyle, l'un des symboles $R^1$, $R^2$ et $R^4$ seulement pouvant être le méthyle et, dans le cas où $R^5$ et/ou $R^6$ représentent le méthyle, $R^1$, $R^2$ et $R^4$ représentent l'hydrogène.

**5.** La hexahydro-2,5,5,8a-tétraméthyl-2H-2,4a-méthano-naphtalèn-3(4H)-one comme composé selon la revendication 4.

**6.** Composés de formule :

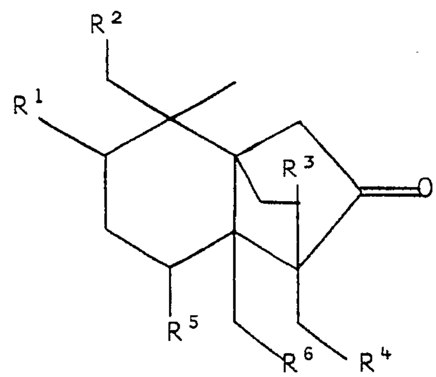

V

où $R^1$ à $R^6$ représentent l'hydrogène ou le méthyle, l'un des symboles $R^1$ à $R^4$ seulement pouvant être le méthyle et, dans le cas où $R^5$ et/ou $R^6$ représentent le méthyle, $R^1$ à $R^4$ représentent l'hydrogène.

**7.** La 1,7a-trans-hexanydro-1,4,4,7a-tétraméthyl-1,3a-éthano-3aH-indèn-2(3H)-one comme composé selon la revendication 6.

**8.** Composés de formule :

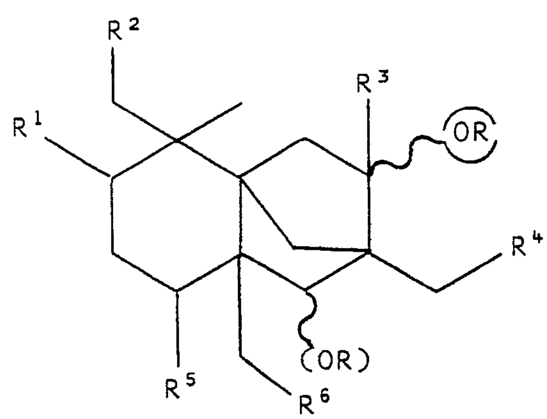

III

où l'un des symboles $R^1$ à $R^6$ représentent l'hydrogène ou le méthyle, l'un des symboles $R^1$ à $R^4$ seulement pouvant être le méthyle et, dans le cas où $R^5$ et/ou $R^6$ représentent le méthyle, $R^1$, $R^2$, $R^3$ et $R^4$ représentent l'hydrogène et R représente l'hydrogène, le formyle ou l'acétyle et les parenthèses () signifient qu'un reste OR seulement est présent dans la molécule.

**9.** Composition odoriférante, caractérisée en ce qu'elle contient un composé de formule :

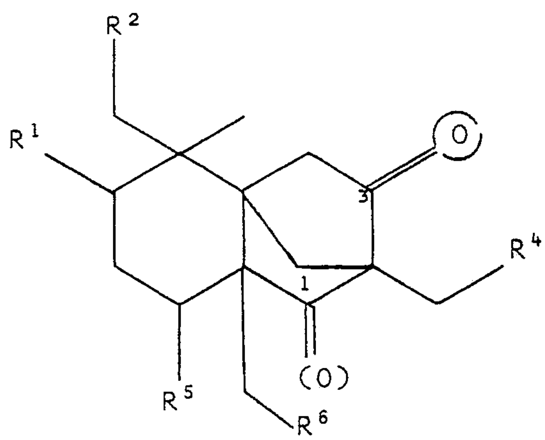

I

où les symboles $R^1$ , $R^2$, $R^4$, $R^5$ et $R^6$ représentent l'hydrogène ou le méthyle, l'un des symboles $R^1$, $R^2$ et $R^4$ seulement pouvant être le méthyle et, dans le cas où $R^5$ et/ou $R^6$ représentent le méthyle, $R^1$, $R^2$ et $R^4$ représentent l'hydrogène et les parenthèses () signifient qu'un groupe céto seulement est présent dans la molécule et, dans le cas d'un groupe céto en 1, l'atome de carbone en 3 peut être également monométhyle substitué.

**10.** Composition odoriférante selon la revendication 9, caractérisée en ce qu'elle contient un composé I où $R^1$ = $R^2$ = $R^4$ = $R^5$ = $R^6$ = hydrogène.

**11.** Procédé pour la préparation des composés de formule :

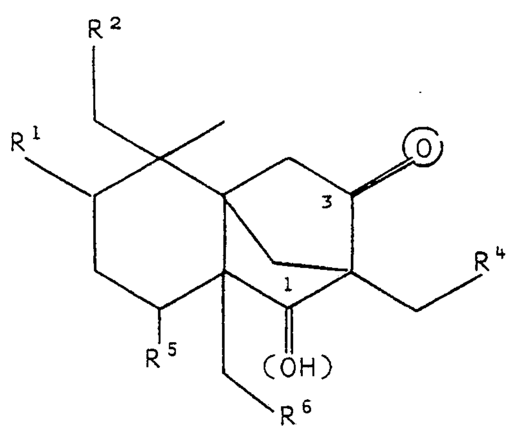

I

où les symboles $R^1$, $R^2$, $R^4$ , $R^5$ et $R^6$ représentent l'hydrogène ou le méthyle, l'un des symboles $R^1$ , $R^2$ et $R^4$ seulement pouvant être le méthyle et, dans le cas où $R^5$ et/ou $R^6$ représentent le méthyle, $R^1$ , $R^2$ et $R^4$ représentent l'hydrogène et les parenthèses () signifient qu'un groupe céto seulement est présent dans la molécule et, dans le cas d'un groupe céto en 1, l'atome de carbone en 3 peut être monométhyle substitué, caractérisé en ce que l'on oxyde un composé de formule :

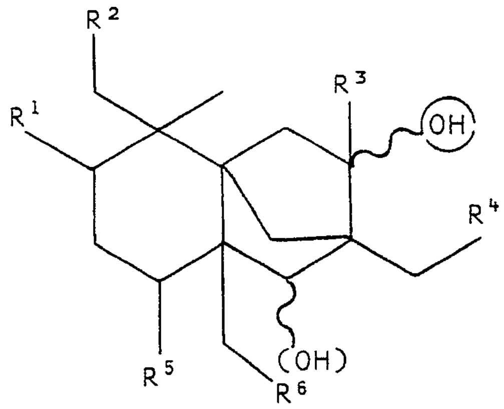

II

où les parenthèses signifient qu'un groupe hydroxy seulement est présent dans la molécule et $R^3$ est l'hydrogène ou le méthyle.

**12.** Procédé selon la revendication 11, caractérisé en ce que l'on oxyde un composé de formule II où $R^1 = R^2 = R^3 = R^4 = R^5 = R^6$ = hydrogène.

**13.** Procédé pour la préparation de compositions odoriférantes, caractérisé en ce que l'on associe un composé I selon la revendication 1 avec les adjuvants de formulation usuels et éventuellement avec d'autres substances odoriférantes.

**14.** Utilisation des composés de formule :

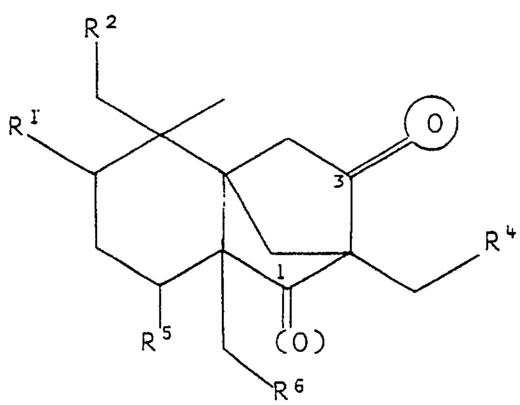

I

où les symboles $R^1$, $R^2$, $R^4$, $R^5$ et $R^6$ représentent l'hydrogène ou le méthyle, l'un des symboles $R^1$, $R^2$ et $R^4$ seulement pouvant être le méthyle et, dans le cas où $R^5$ et/ou $R^6$ représentent le méthyle, $R^1$, $R^2$ et $R^4$ représentent l'hydrogène et les parenthèses () signifient qu'un groupe céto seulement est présent dans la molécule et, dans le cas d'un groupe céto en 1, l'atome de carbone en 3 peut être également monométhyle substitué, comme substance odoriférante.